# EUROPÄISCHE PATENTSCHRIFT

(11) **EP 1 113 284 B1**
(45) Veröffentlichungstag und Bekanntmachung des Hinweises auf die Patenterteilung: **14.12.2005**
(21) Anmeldenummer: 00204061.6
(22) Anmeldetag: 16.11.2000
(51) Int. Cl.: G01R 33/28, A61B 5/055

(54) **MR-Verfahren zur Anregung der Kernmagnetisierung in einem begrenzten räumlichen Bereich**
MR method of excitation of nuclear magnetization in a limited spatial region
Procédé RM d'excitation de la magnétisation nucléaire dans une région spatiale limitée

(30) Priorität: 25.11.1999 DE 19956595
(43) Veröffentlichungstag der Anmeldung: 04.07.2001
(73) Patentinhaber: Philips Intellectual Property & Standards GmbH, 20099 Hamburg (DE); Koninklijke Philips Electronics N.V., 5621 BA Eindhoven (NL)
(72) Erfinder: Weiss, Steffen, Philips Corporate, 52064 Aachen (DE); Lüdeke, Kai-Michael, Philips Corporate, 52064 Aachen (DE)
(74) Vertreter: Volmer, Georg

(56) Entgegenhaltungen:
- EP-A- 0 928 972
- WO-A-99/19739
- DE-A- 3 500 456

## Beschreibung

Die Erfindung betrifft ein MR-Verfahren (MR=Magnetresonanz) zur Anregung der Kernmagnetisierung in einem begrenzten räumlichen Bereich eines Untersuchungsobjektes mit einer in diesem Bereich befindlichen Mikrospule, auf die wenigstens ein Hochfrequenz-Impuls einwirkt. Die Erfindung betrifft ferner eine Vorrichtung zur Durchführung des Verfahrens nach Anspruch 1.

Ein Verfahren und eine Vorrichtung dieser Art sind aus der EP-A 928 972 (PHD 98-002) bekannt. Der Mikrospule ist dabei eine Kapazität parallelgeschaltet, so daß ein im wesentlichen auf die Frequenz der HF-Impulse (HF=Hochfrequenz) abgestimmter Resonanzkreis entsteht. Das durch die HF-Impulse erzeugte, das Untersuchungsobjekt durchsetzende (äußere) Magnetfeld wird im Nahbereich der Mikrospule verstärkt und in seiner Phase verschoben. Dies führt wiederum dazu, daß die Kernmagnetisierung in diesem Bereich verstärkt angeregt wird, so daß sich dieser Nahbereich in dem MR-Bild gegenüber den anderen Bereichen des Untersuchungsobjektes abhebt.

Eine ähnliche Vorrichtung ist aus der WO 99/19739 bekannt, welche eine Mikrospule für ein medizinisches Instrument offenbart, die zu einem nicht-linearen Resonanzkreis mittels antiparallel geschalteter Dioden verschaltet ist.

Die Anregung der Kernmagnetisierung in einem begrenzten Bereich läßt sich auf verschiedene Weise ausnutzen: Eine Möglichkeit besteht darin, ein MR-Bild dieses eng begrenzten Bereiches - z.B eines Blutgefässes - zu erzeugen. Die Beschränkung des MR-Bildes auf einen kleinen Bereich führt zu kurzen Meßzeiten und gestattet fluoroskopische Anwendungen. - Eine andere Möglichkeit besteht in der Lokalisierung der Mikrospule und ggf. eines damit verbundenen Instrumentes. Dabei wird das z.B. medizinische Instrument an der zu markierenden Stelle mit einer Mikrospule ausgestattet.

Die Akquisition der MR-Signale zur Abbildung eines begrenzten Bereiches bzw. zur Lokalisierung der Mikrospule können zwar relativ einfach und schnell durchgeführt werden. Jedoch werden dabei MR-Signale aus dem gesamten Bereich empfangen, in dem die Bedingungen für eine Magnetresonanz erfüllt sind. Dieser Bereich ist wesentlich größer als der (Nah-) Bereich, in dem sich das Magnetfeld der Mikrospule im wesentlichen konzentriert. Dadurch ergeben sich bei den MR-Bildern des Nahbereiches Aliasing-Artefakte; bei der Lokalisierung mittels dreier orthogonaler Projektionsmessungen resultiert daraus ein Signaluntergrund, der die Lokalisierung eventuell unmöglich macht.

Der Erfindung liegt deshalb die Aufgabe zugrunde, ein Verfahren der eingangs beschrieben Art so auszugestalten, daß die Anregung der Kernmagnetisierung besser auf den Nahbereich der Mikrospule beschränkt ist. Weiterhin soll ein MR-Gerät zur Durchführung des Verfahrens geschaffen werden.

Diese Aufgabe wird gemäß Anspruch 1 mit dem genannten Verfahren mit folgenden Schritten gelöst,
- Erzeugung des Hochfrequenz-Impulses mittels einer mit der Mikrospule verbundenen aktiven oder passiven Schaltung mit einem Frequenzspektrum, das sich nicht mit der Larmorfrequenz überlappt, so daß dadurch in dem Untersuchungsobjekt die Kernmagnetisierung nicht angeregt wird,
- Erzeugung eines zusätzlichen Frequenzspektrums durch die Mikrospule unter der Einwirkung des Hochfrequenz-Impulses, das sich mit der Larmorfrequenz überlappt, so daß im Nahbereich der Mikrospule die Kernmagnetisierung angeregt wird.

Außerhalb des Nahbereichs der Mikrospule kann keine Kernmagnetisierung angeregt werden, weil dort wegen des für die Erfindung spezifischen Spektrums des Hochfrequenzimpulses die Bedingungen für eine Magnetresonanz nicht erfüllt sind. Nur im Nahbereich der Mikrospule ist dies infolge Modifikation des Spektrums durch die Mikrospule der Fall. Deshalb kann nur dort eine Kernmagnetiserung angeregt werden. Die Erfindung hat somit den Vorteil, daß in den MR-Signalen praktisch kein aus dem gesamten Bereich stammendes Untergrundsignal vorhanden ist, so daß durch den wesentlich höheren Signal-Kontrast die Probleme der bisher bekannten Methode vermieden werden.

Ein zur Durchführung des erfindungsgemäßen Verfahrens geeignetes MR-Gerät ist in Anspruch 5 angegeben.

Die Ansprüche 2 und 4 geben verschiedene Möglichkeiten zur Informationsgewinnung aus der räumlich begrenzten Anregung der Kernmagnetisierung an. Bei der Ausgestaltung nach Anspruch 3 wird abwechselnd die Kernmagnetisierung im Nahbereich und in einem diesen umschließenden, größeren Bereich angeregt. Dadurch werden zusätzlich die MR-Signale für ein MR-Bild akquiriert, in dem die aus der Lokalisierung resultierende Position bei Bedarf sichtbar gemacht werden kann. Die abwechselnde Akquisition von MR-Signalen zur Bildgebung und zur Lokalisierung ist insbesondere bei bewegten Objekten von Vorteil, weil Bildgebung und Lokalisierung sich nahezu auf den gleichen Zeitraum beziehen. Zur Erhöhung der Bewegungsauflösung kann die Dauer und die Häufigkeit der beiden Vorgänge (das heißt ihr Tastverhältnis) vorteilhaft aufeinander abgestimmt werden.

Der Anspruch 6 beschreibt eine bevorzugte Ausgestaltung der Mikrospule.

Weitere Einzelheiten, Merkmale und Vorteile der Erfindung ergeben sich aus der folgenden Beschreibung einer bevorzugten Ausführungsform anhand der Zeichnung. Es zeigt:
Fig. 1 eine stark vereinfachte Darstellung einer Vorrichtung zur Erzeugung von MR-Bildern;
Fig. 2 ein Prinzipschaltbild zum Betrieb einer solchen Vorrichtung;
Fig. 3 ein Ausführungsbeispiel einer zu einem nichtlinearen Resonanzkreis verschalteten Mikrospule;
Fig. 4 ein Diagramm verschiedener Frequenzverläufe und
Fig. 5 eine schematische Darstellung verschiedener Sequenzen bei einer MR-Bilderzeugung und Lokalisierung einer Mikrospule.

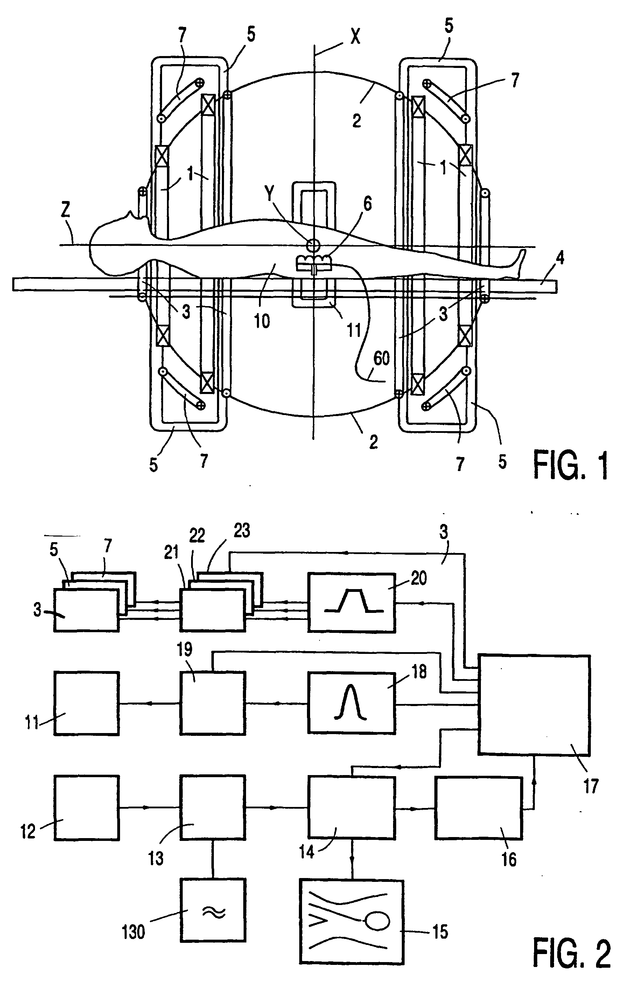

Die in Figur 1 dargestellte Vorrichtung zur Erzeugung von MR-Bildern, die auch als Kernspinuntersuchungsgerät bezeichnet wird, weist eine aus vier Hauptspulen 1 bestehende Anordnung zur Erzeugung eines homogenen stationären Magnetfeldes in z-Richtung (Hauptfeld) auf, dessen magnetische Flußdichte (magnetische Induktion) in der Größenordnung von einigen Zehntel Tesla bis einigen Tesla liegen kann. Die zu der z-Achse konzentrisch angeordneten Hauptspulen 1 können auf einer Kugeloberfläche 2 liegen. Im Innern dieser Spulen befindet sich ein Untersuchungsobjekt, zum Beispiel ein Patient 10 auf einer Tischplatte 4.

Zur Erzeugung eines in Richtung der z-Achse verlaufenden und sich in dieser Richtung linear ändernden ersten Gradienten-Magnetfeldes sind vier erste Spulen 3 auf der Kugeloberfläche 2 oder einer Zylinderoberfläche angeordnet. Weiterhin sind vier zweite Spulen 7 vorgesehen, die ein ebenfalls in Richtung der z-Achse verlaufendes zweites Gradienten-Magnetfeld erzeugen, das sich jedoch in vertikaler Richtung (x-Richtung) linear ändert. Schließlich wird mit vier dritten Spulen 5 (von denen nur zwei dargestellt sind) ein in Richtung der z-Achse verlaufendes, drittes Gradient-Magnetfeld erzeugt, das sich senkrecht zur Zeichenebene der Figur 1 (y-Richtung) linear ändert.

In den zu untersuchenden Bereich des Patienten ist ein medizinisches Instrument (zum Beispiel ein Katheter) 60 eingeführt, an dessen Spitze sich eine Mikrospule L befindet. Dieser Bereich ist ferner von einer durch einen HF-Impuls beaufschlagbaren Hochfrequenz-Sendespule 11 umgeben, durch die dieser Bereich mit einem eine Spinresonanz anregenden HF-Magnetfeld durchsetzt wird. Die sich an diese Anregung anschließende Relaxation bewirkt eine Änderung der Magnetisierungszustände, die in einer Hochfrequenz-Empfangsspule 12 (siehe Figur 2) eine entsprechende Spannung induziert, die zur MR-Bilderzeugung ausgewertet wird, wobei die Gradienten-Magnetfelder eine Lokalisierung der angeregten Zustände ermöglichen.

Die zum Betrieb dieser Vorrichtung wesentlichen Komponenten sind schematisch in Figur 2 dargestellt und umfassen eine Steuereinheit 17, die einen Gradienten-Wellenform-Generator 20 ansteuert, an dessen Ausgängen jeweils ein erster, ein zweiter und ein dritter Gradientenverstärker 21, 22, 23 angeschlossen ist. Diese Verstärker erzeugen jeweils den Strom für die erste, zweite bzw. dritte Spule 3, 5, 7. Die Verstärkungsfaktoren dieser Verstärker sind durch die Steuereinheit 17 über Leitungen 32 unabhängig voneinander einstellbar, so daß die Spulen 3, 5, 7 die Gradientfelder in den x-, y- und z-Richtungen erzeugen und eine Schichtselektion in den entsprechenden drei Raumrichtungen in dem untersuchten Bereich vorgenommen werden kann.

Weiterhin wird durch die Steuereinheit 17 ein HF-Generator 18 angesteuert, um einerseits zur MR-Bilderzeugung die Frequenz der HF-Impulse auf die von den Gradientfeldern abhängigen Larmor-Frequenzen abzustimmen und andererseits zur Lokalisierung der Mikrospule eine Umschaltung der Frequenz in der Weise vorzunehmen, daß eine Magnetresonanz nur noch in deren Nahbereich angeregt wird. Die HF-Impulse werden einem Verstärker 19 zugeführt, dessen Verstärkung durch die Steuereinheit 17 gesteuert wird, und gelangen anschließend zu der Hochfrequenz-Sendespule 11.

Die in der Hochfrequenz-Empfangsspule 12 durch die Relaxation der angeregten Magnetisierungszustände induzierten MR-Signale werden in einem Quadratur-Demodulator 13 durch Mischung mit zwei um 90° gegeneinander versetzten Trägerschwingungen (mit einer durch die lokale Stärke der stationären Magnetfelder bestimmten Larmor- bzw. MR-Frequenz) eines Oszillators 130 demoduliert, so daß zwei Signale entstehen, die als Realteil und als Imaginärteil eines komplexen Signals aufgefaßt werden können. Diese Signale werden einem Analog-Digitalwandler 14 zugeführt. Mit einer Bildverarbeitungseinheit 16 werden schließlich die MR-Bilder in bekannter Weise rekonstruiert und auf einem Monitor 15 wiedergegeben.

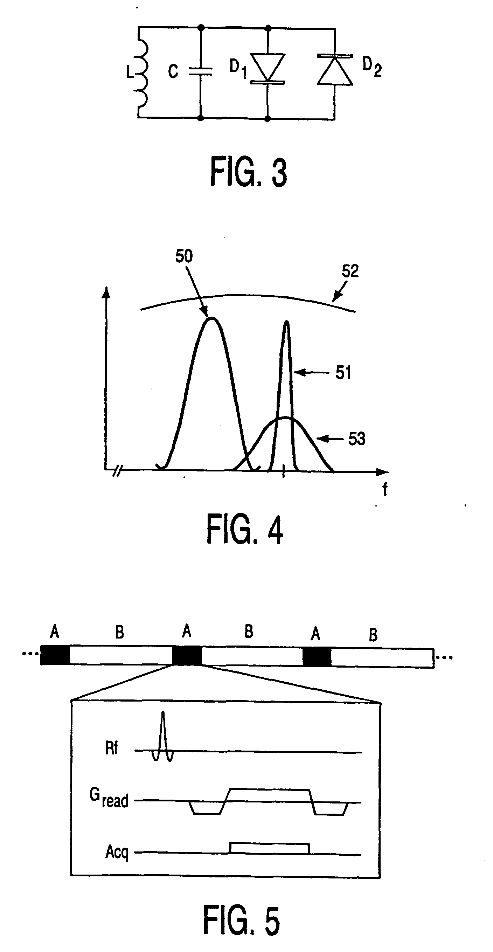

Figur 3 zeigt die Mikrospule L sowie eine beispielhafte Verschaltung dieser Spule zu einem nichtlinearen passiven Resonanzkreis. Dieser Resonanzkreis umfaßt in Parallelschaltung zu der Spule L eine Kapazität C sowie zwei antiparallel geschaltete Dioden D₁, D₂, die die Nichtlinearität erzeugen. Anstelle der Dioden können zu diesem Zweck natürlich auch andere Elemente verwendet werden. Der gesamte Resonanzkreis ist vorzugsweise mit miniaturisierten Bauelementen realisiert, so daß er vollständig an der Spitze eines in den zu untersuchenden Bereich einzuführenden medizinischen Instrumentes 60 angeordnet werden kann und keine nach außen geführten Anschlußleitungen erforderlich sind. Die Mikrospule ist hierbei also passiv wirksam, d.h. es gibt keine von der Mikrospule nach außen geführten Zuleitungen, die immer mit dem Risiko verknüpft sind, daß darin für den Patienten schädliche Spannungen bzw. Ströme induziert werden können.

Durch die in den Resonanzkreis eingeführte Nichtlinearität kann das medizinische Instrument 60, das die Mikrospule L trägt, in dem zu untersuchenden Bereich wesentlich sicherer anhand der nun untergrundfreien Projektions-Meßsignale lokalisiert werden. Dies beruht auf der in Figur 4 dargestellten relativen Lage der verschiedenen Frequenzbereiche. Zur Lokalisierung des Instrumentes 60 wird zunächst mittels der Steuereinheit 17 der HF-Generator 18 so umgeschaltet, daß das Spektrum 50 des HF-Sendeimpulses außerhalb der Spektrums 51 um die Larmorfrequenz 51 liegt, so daß in dem zu untersuchenden Bereich keine zur MR-Bilderzeugung erforderliche ("globale") Spinresonanz angeregt wird. Gleichzeitig liegt das Spektrum 50 des HF-Sendeimpulses jedoch innerhalb des wesentlich breiteren Resonanzfrequenzganges 52 des Resonanzkreises.

Das verschobene Spektrum des HF-Sendeimpulses und die in den Resonanzkreis eingeführte Nichtlinearität sind nun so gewählt, daß durch die Nichtlinearität lokal, d.h. nur im Nahbereich der Mikrospule, ein (in Figur4 idealisiert dargestelltes) Frequenzspektrum 53 erzeugt wird, das auch die Larmorfrequenz bzw. das Spektrum 51 überdeckt. Dieses lokale Anregungsspektrum bzw. der entsprechende, durch den Resonanzkreis fließende Strom reicht aus, um im Nahbereich der Mikrospule eine magnetische Flußdichte zu erzeugen, durch die eine Spinresonanz angeregt wird

Die Lokalisierung dieser Spinresonanz und somit der Mikrospule L bzw. des medizinischen Instruments erfolgt gemäß Figur 5 durch Erfassen der aus der Anregung resultierenden MR-Signale im wesentlichen in der Weise, wie auch die MR-Bilderzeugung nach Anregung der Spinresonanz im gesamten zu untersuchenden Bereich vorgenommen wird. Im Falle einer dreidimensionalen Lokalisierung kann eine Spin- oder Gradienten-Echo-Signalauswertung erfolgen, wobei vorzugsweise keine Schichtselektionen und keine Phasenkodierung erfolgen, sondern lediglich ein Auslese-Gradient-Magnetfeld in einer der drei Raumrichtungen (frequenzcodierendes Magnetfeld) angelegt wird, um das aufgenommene Signal durch Fourier-Transformation im Hinblick auf die Amplituden der Frequenzkomponenten entlang der betreffenden Richtung auszuwerten. Diese Messung und Auswertung wird im Wechsel für alle drei Raumrichtungen und im Wechsel mit der Messung für die MR-Bilderzeugung durchgeführt (bei der das Spektrum des Hochfrequenzimpulses die Larmorfrequenz in dem abzubildenden Bereich überdeckt), um quasi simultan die dreidimensionale Position zu bestimmen und das MR-Bild zu erzeugen. Alternativ zu dem beschriebenen Projektionsverfahren kann die Messung der im Nahbereich der Mikrospule angeregten Kernmagnetisierung auch durch ein beliebiges anderes Verfahren erfolgen.

Figur 5 zeigt hierzu beispielhaft einen frequenzverschobenen HF-lmpuls Rf und darunter den Verlauf eines Gradienten-Magnetfeldes G_{read} zur Gradienten-Echo-Signalauswertung sowie ein ausgewertetes Signal Acq zur Lokalisierung.

Wie aus Figur 5 weiter deutlich wird, können die Vorgänge A zur Lokalisierung der Mikrospule und B zur MR-Bilderzeugung des Untersuchungsobjektes abwechselnd durchgeführt werden, so daß sich zwei ineinander verschachtelte Sequenzen A, B, A, B,.. ergeben. Die Länge der beiden Vorgänge A, B, das heißt die Folgefrequenz der Sequenzen sowie deren Tastverhältnis können in Abhängigkeit von der betreffenden Anwendung, insbesondere der Bewegungsgeschwindigkeit der Mikrospule, des darzustellenden Mediums sowie der gewünschten Bewegungsauflösung nahezu frei gewählt werden. Die Lokalisierung kann zum Beispiel 6 ms dauern, während für die MR-Bilderzeugung 500 ms angesetzt werden.

Weiterhin kann während eines Lokalisierungsvorgangs entweder eine vollständige dreidimensionale Bestimmung der Position der Mikrospule vorgenommen werden, oder es wird mit jedem Vorgang die Lage in nur einer Richtung bestimmt, so daß sich erst nach drei Lokalisierungsvorgängen die Position der Mikrospule ergibt.

Im Falle der Benutzung der Mikrospule zur Bildgebung eines eng begrenzten Bereichs -z.B eines Blutgefässes - wird ebenfalls ein HF-Sendepuls mit frequenzverschobenem Spektrum verwendet in Kombination mit einem beliebigen bildgebenden MR-Verfahren, das der Bildgebung in einem eng begrenzten Bereich angepaßt ist.

## Patentansprüche

1. MR-Verfahren zur Anregung der Kernmagnetisierung in einem begrenzten räumlichen Bereich eines Untersuchungsobjektes mit einer in diesem Bereich befindlichen Mikrospule, auf die wenigstens ein Hochfrequenz-Impuls einwirkt,
**gekennzeichnet durch** folgende Schritte:
Erzeugung des Hochfrequenz-Impulses mit einem Frequenzspektrum, das sich nicht mit der Larmorfrequenz überlappt, so daß **dadurch** in dem Untersuchungsobjekt die Kernmagnetisierung nicht angeregt wird, wobei die Mikrospule mit einer aktiven oder passiven Schaltung verbunden ist, so daß ein
zusätzliches Frequenzspektrum **durch** die Mikrospule unter der Einwirkung des Hochfrequenz-Impulses erzeugt wird, das sich mit der Larmorfrequenz überlappt, so daß im Nahbereich der Mikrospule die Kernmagnetisierung angeregt wird.

2. MR-Verfahren nach Anspruch 1,
**gekennzeichnet durch**
seine Anwendung zur Lokalisierung der Mikrospule.

3. MR-Verfahren nach Anspruch 2, **gekennzeichnet dadurch, daß** zusätzlich eine Reihe von Sequenzen zwecks Gewinnung eines MR-Bildes von einem Bereich des Untersuchungsobjektes erzeugt wird, der größer ist als der Nahbereich, und
daß die zur Lokalisierung der Mikrospule (L) erforderlichen Messungen abwechselnd mit der auf den Nahbereich der Mikrospule beschränkten Anregung der Kernmagnetisierung erfolgen.

4. MR-Verfahren nach Anspruch 1,
**dadurch gekennzeichnet, daß** die im Nahbereich der Mikrospule (L) angeregte Kernmagnetisierung in einem MR-Bild dargestellt wird.

5. MR-Gerät zur Durchführung des Verfahrens nach Anspruch 1 mit einem Magneten (1) zur Erzeugung eines homogenen stationären Magnetfeldes, dessen Stärke die Larmorfrequenz definiert, mit Mitteln (11) zur Erzeugung von Hochfrequenz-Impulsen, Mitteln (12) zum Empfangen von im Untersuchungsobjekt erzeugten MR-Signalen und einer Mikrospule,
**gekennzeichnet durch** eine Steuereinrichtung (17) zum Steuern des Frequenzspektrums des Hochfrequenz-Impulses in der Weise, daß **dadurch** in dem Untersuchungsobjekt keine Kernmagnetisierung angeregt wird, daß das Frequenzspektrum des Hochfrequenz-Impulses mittels einer mit der Mikrospule verbundenen aktiven oder passiven Schaltung in deren Nahbereich so verändert wird, daß es dort die Larmorfrequenz überdeckt und daß die im Nahbereich der Mikrospule entstehenden MR-Signale von den Mitteln (12) zum Empfangen erfaßbar sind.

6. MR - gerät nach Anspruch 5
**dadurch gekennzeichnet,**
**daß** die Mikrospule zu einem nichtlinearen Resonanzkreis verschaltet ist.

7. MR - gerät nach Anspruch 6
**dadurch gekennzeichnet,**
**daß** der Resonanzkreis zwei antiparallel geschaltete Dioden (D₁, D₂) aufweist, die dessen Nichtlinearität erzeugen.

8. MR- gerät nach Anspruch 6
**dadurch gekennzeichnet,**
**daß** es außerdem ein in einen Patienten einführbases medizinisches Instrument (60) aufweist, an dem der Resonanzkreis in Form eines miniaturisierten Schaltungsmoduls befestigt ist.

## Claims

1. An MR method for exciting the nuclear magnetization in a limited spatial region of an object to be examined, utilizing a microcoil which is present in said region and is subject to at least one RF pulse, **characterized in that** the method includes the following steps:
generating the RF pulse which has a frequency spectrum that does not overlap the Larmor frequency, so that the nuclear magnetization in the object to be examined is not excited thereby, the microcoil being connected to an active or passive circuit so that an additional frequency spectrum is generated by the microcoil under the influence of the RF pulse, which additional frequency spectrum overlaps the Larmor frequency in such a manner that the nuclear magnetization is excited in the close range of the microcoil.

2. An MR method as claimed in claim 1, **characterized in that** it is used to localize the microcoil.

3. An MR method as claimed in claim 2, **characterized in that** additionally a series of sequences is generated in order to acquire an MR image of a region of the object to be examined which is larger than the close range, and **in that** the measurements required for the localization of the microcoil (L) are performed so as to alternate with the excitation of the nuclear magnetization which is restricted to the close range of the microcoil.

4. An MR method as claimed in claim 1, **characterized in that** the nuclear magnetization excited in the close range of the microcoil (L) is reproduced in an MR image.

5. An MR apparatus for carrying out the method as claimed in claim 1, including a magnet (1) for generating a uniform, steady magnetic field whose strength defines the Larmor frequency, means (11) for generating RF pulses, and means (12) for receiving MR signals generated in the object to be examined, **characterized in that** it includes a control device (17) for controlling the frequency spectrum of the RF pulses in such a manner that no nuclear magnetization is excited thereby in the object to be examined, **in that** the frequency spectrum of the RF pulse is modified, utilizing an active or passive circuit connected to the microcoil in the close range of the microcoil in such a manner that it overlaps the Larmor frequency, and **in that** MR signals arising in the close range of the microcoil can be received by the receiving means (12).

6. An MR apparatus as claimed in claim 5, **characterized in that** the microcoil is wired so as to form a non-linear resonant circuit.

7. An MR apparatus as claimed in claim 6, **characterized in that** the resonant circuit includes two diodes (D₁, D₂) connected in anti-parallel which induce its nonlinearity.

8. An MR apparatus as claimed in claim 6, **characterized in that** it additionally includes a medical instrument (60) that can be introduced into a patient, to which instrument the resonant circuit is attached in the form of a miniaturized circuit module.

## Revendications

1. Procédé RM d'excitation de la magnétisation nucléaire dans une zone spatiale délimitée d'un objet à examiner avec une microbobine se trouvant dans cette zone sur laquelle agit au moins une impulsion à haute fréquence,
**caractérisé par** les étapes suivantes:
production de l'impulsion à haute fréquence avec un spectre de fréquence qui ne chevauche pas la fréquence de Larmor de telle sorte que, de ce fait, la magnétisation nucléaire ne soit pas excitée dans l'objet à examiner, la microbobine étant reliée avec un circuit actif ou passif de telle sorte qu'un spectre de fréquence supplémentaire soit produit par la microbobine sous l'action de l'impulsion à haute fréquence qui se superpose à la fréquence de Larmor de telle sorte que la magnétisation nucléaire soit excitée dans la zone de proximité de la microbobine.

2. Procédé RM selon la revendication 1,
**caractérisé par**
son application pour la localisation de la microbobine.

3. Procédé RM selon la revendication 2, **caractérisé en ce qu'**en plus, une série de séquences est produite pour la production d'une image RM d'une zone de l'objet à examiner qui soit supérieure à la zone de proximité et que les mesures nécessaires pour la localisation de la microbobine (L) sont effectuées en alternance avec l'excitation de la magnétisation nucléaire limitée à la zone de proximité de la microbobine.

4. Procédé RM selon la revendication 1,
**caractérisé en ce que** la magnétisation nucléaire excitée dans la zone de proximité de la microbobine (L) est représentée dans une image RM.

5. Appareil RM pour l'exécution du procédé selon la revendication 1 avec un aimant (1) pour la production d'un champ magnétique stationnaire homogène dont l'intensité définit la fréquence de Larmor, avec des moyens (11) pour la production d'impulsions à haute fréquence, des moyens (12) pour la réception de signaux RM produits dans l'objet à examiner et une microbobine,
**caractérisé par** un dispositif de commande (17) pour la commande du spectre de fréquence de l'impulsion à haute fréquence de telle manière qu'aucune magnétisation nucléaire ne soit excitée de cette manière dans l'objet à examiner, que le spectre de fréquence de l'impulsion à haute fréquence soit modifié à l'aide d'un circuit actif ou passif relié à la microbobine dans sa zone de proximité de telle sorte qu'il y recouvre la fréquence de Larmor et que les signaux RM formés dans la zone de proximité de la microbobine puissent être enregistrés par les moyens (12) pour la réception.

6. Appareil RM selon la revendication 5, **caractérisé en ce que** la microbobine est montée en un circuit de résonance non linéaire.

7. Appareil RM selon la revendication 6, **caractérisé en ce que** le circuit de résonance présente deux diodes (D1, D2) en montage antiparallèle qui produisent sa non-linéarité.

8. Appareil RM selon la revendication 6, **caractérisé en ce qu'**il présente par ailleurs un instrument médical (60) à introduire dans un patient, auquel le circuit de résonance est fixé sous la forme d'un module de circuit miniaturisé.
